# EUROPEAN PATENT SPECIFICATION

(11) **EP 4 265 635 B1**
(45) Date of publication and mention of the grant of the patent: **03.12.2025**
(21) Application number: 21907196.6
(22) Date of filing: 11.03.2021
(51) Int. Cl.: C07K 14/78, C07K 1/36, A61K 38/39, A61L 27/24, A61L 27/36

(54) **METHOD FOR PRODUCING HUMAN COLLAGEN STRUCTURES WITH CONTROLLED CHARACTERISTICS**
VERFAHREN ZUR HERSTELLUNG VON MENSCHLICHEN KOLLAGENSTRUKTUREN MIT KONTROLLIERTEN EIGENSCHAFTEN
PROCÉDÉ POUR PRODUIRE DES STRUCTURES DE COLLAGÈNE HUMAIN AYANT DES CARACTÉRISQUES CONTRÔLÉES

(30) Priority: 18.12.2020 MX 2020014328
(43) Date of publication of application: 25.10.2023
(73) Proprietor: Ortega Blanco, José Adán, Jalisco, 45070 (MX)
(72) Inventor: AGUILAR ALEMÁN, Juan Pablo, Jalisco, 45070 (MX); CAMACHO PÉREZ, Beni, Jalisco, 45070 (MX); AGUILLÓN ESTRADA, Brenda Karen, Jalisco, 45070 (MX); CARDOSO HERNÁNDEZ, Grecia Andrea, Jalisco, 45070 (MX); ROJAS GARCÍA, Octavio Israel, Jalisco, 45070 (MX); MIRAMONTES BEAS, Eduardo, Jalisco, 45070 (MX)
(74) Representative: Temiño Ceniceros, Ignacio
(86) International application number: PCT/MX2021/000007
(87) International publication number: WO 2022/131896

(56) References cited:
- EP-A1- 0 431 479
- CN-A- 107 236 777
- CN-A- 110 564 802
- US-A- 4 389 487
- OLIVEIRA VAGNE DE MELO; ASSIS CAIO RODRIGO DIAS; COSTA BEATRIZ DE AQUINO MARQUES; NERI ROBSON COELHO DE ARAúJO; MONTE FL&#225: "Physical, biochemical, densitometric and spectroscopic techniques for characterization collagen from alternative sources: A review based on the sustainable valorization of aquatic by-products", JOURNAL OF MOLECULAR STRUCTURE, ELSEVIER AMSTERDAM, NL, vol. 1224, 5 August 2020 (2020-08-05), NL , XP086376830, ISSN: 0022-2860, DOI: 10.1016/j.molstruc.2020.129023

## Description

### FIELD OF THE INVENTION

The present invention is related to the field of biotechnology in general; in particular, it is related to a novel method for producing human collagen structures with controlled characteristics, which allows good performance while preserving structural integrity and high purity.

### BACKGROUND OF THE INVENTION

Collagen is one of the most abundant essential proteins in the human body, it represents 25% of the total body protein, which is part of different tissues: it provides flexibility, elasticity and resistance to pressure.

This protein is very abundant in skin, bones, connective tissues and joints (ligaments, tendons and cartilage). It is also found in the cornea and in the walls of blood vessels. Collagen is an essential protein for the proper functioning of the body.

Type I collagen is by far the most abundant collagen in the body. It has an unusual amino acid composition, with 33% glycine and 10% proline. It also has 0.5% 3-hydroxyproline, 10% 4-hydroxyproline, and 1% 5-hydrolysine. These hydroxylated amino acids are post-translationally synthesized from prolyl and lysyl residues in the polypeptide. Collagen has a small amount of carbohydrates, most of them attached to the hydroxyl group of hydroxyl isine in the form of Glu-Gal disaccharide. Carbohydrate content in fibrillar collagens is low (0.5-1% in types I and II), but is higher in some non-fibrillar types (14% in type IV). Meisenberg, G. and Simmmon, W. Principles of Medical Biochemistry, 4th Edition.

Type I collagen has the special feature of providing flexibility and elasticity, it is the most abundant in the body, mainly in the bones, tendons, skin and cornea; while type II collagen provides resistance to pressure and is mainly located in cartilage.

Collagen is used extensively in cosmetic surgery and in the construction of artificial skin grafts for the treatment of patients with severe burns. Collagen products are also used in orthopedic and surgical applications in a wide range of dental procedures. Human collagen is derived from cadaveric tissues or debris from surgical procedures, such as the placenta at the time of childbirth; and it has the advantage of significantly reducing the probability of an immune reaction, compared to collagen from other species.

Due to the importance of collagen in several medical and industrial applications, processes have been developed to produce human collagen structures.

There are industrial processes that can produce similar results in terms of obtaining collagen. However, no process has been found that can specifically obtain type I human collagen, with a yield of 35%. Nor has a process been found that can transform the human collagen obtained in varied two-dimensional or three-dimensional structures with controllable characteristics. Nor has a process been found that can precisely control the characteristics of collagen.

Other processes for obtaining collagen are also known that comprise defined stages already widely known by those who are engaged in this area. The stages, in general, include a pretreatment of raw material, hydrolysis and purification of collagen.

There are processes in which certain operating parameters or stages have been slightly modified to extract collagen from several animal sources, such as cattle, pigs, horses, birds and marine animals; using the skin, scales, bones, tendons, pericardium, cartilage, etc., of said animals.

Methods of collagen production in vitro, through the use of cell lines, are also known. Other known methodologies describe an acidic or basic pretreatment of raw material, for example, with NaOH at different concentrations, temperatures and exposure times. There are also hydrolysis techniques for obtaining soluble collagen in several acids, or through enzymatic treatments. Organic acids such as acetic acid, lactic acid, citric acid and the like have been used for chemical hydrolysis; or inorganic acids such as hydrochloric acid; while proteolytic enzymes such as ficin, pepsin, among others, are reported for enzymatic hydrolysis. Similarly, these techniques are operated in various ranges of operating parameters of temperature, time, pH and concentration of the solution and enzyme.

Other processes are those designed to obtain and purify several types of collagen from the same source, by means of fractional precipitation techniques, at different concentrations of salts such as NaCl, ammonium sulfate and the like, or with ethanol. Methods for collagen purification and concentration, such as dialysis and lyophilization, are also known.

There are methods for producing defined structures of collagen, such as scaffolds, sponges, membranes, coatings, hydrogels, etc., through lyophilization, gelation, electrospinning, bioprinting, among others.

Physical and chemical methods have been described for reinforcing the biopolymer chains and improving the stability and rigidity of the structure, as well as for controlling the degradation rate in vivo and in vitro. As regards these methods, we can mention dehydrothermal treatment, ultraviolet radiation, chemical crosslinking with aldehydes such as glutaraldehyde and formaldehyde in solution, the use of carbodiimides and microbial transglutaminase.

Similarly, there are compression processes for collagen structures, particularly hydrogels, to modify the mechanical properties and fibrillar density of the collagen structure.

The present invention is focused on a novel process for the production of human collagen structures with controlled characteristics, which allows good performance while preserving structural integrity and high purity.

A search was carried out to determine the closest state of the art, and the following documents were found.

Patent document EP0431479 discloses a process for preparing a composition comprising preparing a collagen layer and a chitin layer and fixing them to each other, comprising the steps of tissue conditioning, extraction by enzymatic hydrolysis, precipitation, lyophilization, dialysis, and crosslinking, but not the specific conditions of the present invention, particularly, this document does not mention a moulding nor a second lyophilization step.

Patent document US4389487 was found; this document is by Ries; Peter E. of June 08, 1981, and it discloses a process for the preparation of collagen products for medical and cosmetic use that comprises the stages of finely grinding animal tissues containing collagen at temperatures not exceeding 40 °C, degreasing the resulting tissue pulp and simultaneously removing undesirable components thereof by repeatedly treating said tissue pulp with approximately a five-fold volume, based on pulp volume, of a 5 to 15% aqueous solution of sodium chloride containing about 0.2 to 1 part by weight of sodium azide as a preservative per 1000 parts by weight of said solution and 0.5 to 2% by weight of a non-ionic fat-dispersing wetting agent, washing the resulting fiber pulp with water or with 0.1 to 0.5% aqueous formic, acetic or citric acid, digesting the fiber pulp for 8 to 48 hours at a pH of about 2.5 to 3.5 in a five-fold volume, based on the pulp volume, of 0.1 to 5% of aqueous acetic acid containing about 1 part by weight of pepsin per 1000 parts by weight of tissue used as a starting material for removing non-collagenous proteins and telopeptides, precipitating collagen from the resulting collagen suspension by the addition of aqueous sodium chloride in an amount such that the sodium chloride concentration of the suspension is approximately 3 to 5%, separating the precipitated collagen and substantially desalting it by ultrafiltration, dialysis or washing with 60 to 75% aqueous ethyl alcohol so that the collagen concentration in the desalted solution is approximately 1%, dissolve the desalted collagen in demineralized or distilled water containing up to 3% by weight of a volatile strong organic acid in such a proportion that the collagen concentration in the resulting solution corresponds to a dry residue of approximately 0.5 to 2% by weight, freeze-dry the collagen solution and sterilize the lyophilized collagen product, the improvement comprising: subjecting the collagen product to a heat treatment or gaseous hydrogen halide treatment after lyophilization, before or after sterilization, for a time sufficient to impart increased absorption and wet strength of the collagen product.

The objective of patent US4389487 is the improvement of the physicochemical and mechanical properties of collagen products for their handling during use. In addition, increasing the absorption capacity. The use of a hydrogen chloride atmosphere in the treatment of a collagen product managed to improve the physicochemical and mechanical properties, adding that an absorption of 50 to 100 times its weight was obtained. Compared with the methodology proposed in our invention is the use of a controlled atmosphere of paraformaldehyde to improve physicochemical properties; thus, an absorption of 60 times its weight was achieved. In addition, it should be noted that the proposal of this application is the use of scraps of tendons, fascia lata, amniotic membrane, fat or skin of human origin compared to patent US4389487 where one of its claims is the use of pig skin and bovine tendons.

Patent US4389487 as described above, discloses a process for the preparation of collagen products for medical and cosmetic use that is highly complex, it requires many reagents and variable controls in different percentages; furthermore, the resulting collagen product is subjected to a heat treatment or gaseous hydrogen halide treatment after lyophilization, before or after sterilization, for a time sufficient to impart increased absorption and wet strength of the collagen product.

Document WO2016071876 by Fontanilla Duque, Martha Raquel, et al. of November 06, 2015, was also found; this document discloses a method to isolate type I collagen from the rat tail and produce multidirectional scaffolds (Pérez et al., 2001). In recent years, the isolation method has been modified and standardized. Likewise, changes have been introduced in the method of obtaining scaffolds from this collagen and a novel method has been developed to produce unidirectional lamellar scaffolds. In the optimized method, the animal collagen source is cleaned, cut and suspended in an acetic acid solution until a liquid phase pH between 2.5 and 3.2 is reached, stirring constantly at 4 °C. Said suspension is centrifuged and the pH of the supernatant obtained is neutralized with NaOH to separate the solid and liquid phases again. Collagen precipitated in this way is reprecipitated with salts and solubilized in acetic acid, and the solution is poured into molds, frozen at -20°C and lyophilized. Finally, the scaffolds obtained are crosslinked and then sterilized with ethylene oxide.

The process for obtaining rat collagen is a chemical process compared to the method proposed in our application, which integrates chemical and biological processes (use of pepsin) to obtain the collagen scaffold of human origin. Another difference, in patent WO2016071876, the crosslinking process uses different crosslinking agents (genipin, glutaraldehyde, dendrimers, carbodiimides or a mixture of them) compared to paraformaldehyde, which is the crosslinking agent in our invention. Furthermore, they use ethylene oxide to carry out the final sterilization compared to the process of our invention which uses gamma irradiation.

On the one hand, with the collagen extraction processes described in the literature, although they explore the use of different collagen sources, in the vast majority it is not possible to obtain profitable extraction yields under large-scale conditions, or they are not available in great demand; the most used raw materials for the industrial production of collagen are usually bovine or porcine tissues. However, collagen structures derived from these sources present the risk of eliciting an immune response during their application, since, being a xenograft, biocompatibility decreases.

On the other hand, the possibility of elaborating collagen structures with defined and very specific structural and dimensional characteristics, which contribute to a better integration of the structures in an in vivo environment, influencing cell behavior, has been addressed through techniques that can be very expensive, not very accessible or adaptable on a large scale, or that require specialized equipment, such as electrospinning.

Similarly, despite the fact that collagen is an excellent structural protein and has the advantages of being biocompatible and resorbable, its mechanical properties regarding synthetic polymers are poor. Various methods and techniques have been developed to improve these features and diversify the use of collagen structures; however, said techniques have important disadvantages, such as not being efficient, being processes that require a lot of time, but if they are extended too much, they can denature collagen; some crosslinking techniques or agents can cause a crosslinking gradient along the entire collagen structure, generating a structure with heterogeneous mechanical properties; the addition of some chemical components can result in a cytotoxic effect, and said compounds can remain in the collagen structure even after extensive washing; to mention a few.

### OBJECTIVES OF THE INVENTION

The main objective of the present invention is to make available a process to produce human collagen structures with controlled characteristics that allows to obtain type I human collagen, with a yield of 35%, preserving structural integrity and with purity ≥ 95%. Another objective of the invention is to provide said process for producing human collagen structures with controlled characteristics, which also offers the possibility of producing collagen structures with defined and very specific structural and dimensional characteristics, which contribute to a better integration of the structures in a in vivo environment, influencing cell behavior.

Another objective of the invention is to provide said process to produce human collagen structures with controlled characteristics, which also allows the use of an available, accessible raw material with the highest degree of biocompatibility; obtaining functional and structurally intact collagen, with profitable extraction yields through a compatible process for industrial production conditions.

Another objective of the invention is to provide said process to produce human collagen structures with controlled characteristics, which also allows the elaboration of collagen structures with adjustable physical characteristics for a wide range of biological applications, by means of simple and low-cost techniques that allow the optimization of collagen concentration in the structures; such characteristics include the dimensions of the structure, fibrillar density, porosity and pore size, which strongly influence cell behavior. Another objective of the invention is to provide said process for producing human collagen structures with controlled characteristics, which also allow the mechanical performance of the structures to be increased, with a precise and controlled technique, without affecting the integrity, functionality, or biological safety of collagen.

Another objective of the invention is to provide said process to produce human collagen structures with controlled characteristics, which allows it to be a support or vehicle for the culture of primary and line eukaryotic cells, as well as a deposit for growth factors, proteins and exosomes, for possible use in regenerative medicine.

And all those qualities and objectives that will become apparent when making a general and detailed description of the present invention supported by the illustrated modalities.

### BRIEF DESCRIPTION OF THE INVENTION

The method for producing or process to produce human collagen structures with controlled characteristics ranges from isolating tissue to obtaining collagen, as well as its subsequent transformation into bi- or three-dimensional structures that can be applied in multiple medical procedures. It consists of a total of 8 main stages: tissue conditioning, pre-treatment, extraction, precipitation, dialysis, lyophilization, molding and crosslinking, as well as an optional compression stage.

Likewise, it is intended to protect the entire process itself. From the extraction to the adaptation of the substrate (collagen) to a regenerative device. Our process allows the extraction of allogeneic type 1 collagen, and thereby it can be chemically conditioned (cross-linked) to achieve in vivo functional characteristics. Finally, this last process allows it to be adapted to be able to get ready and thereby achieve the desirable in vitro and in vivo degradation conditions.

In general terms, the process to produce human collagen structures with controlled characteristics, in accordance with the present invention, consists of the following stages:
a) Tissue conditioning, where tissues or fluids that are not of interest for the process are removed and it is reduced to a particle size of 0.5 to 1 mm in a uniform way, in order to achieve a better interaction between solutions and tissue in the subsequent steps.
b) Pre-treatment
   Exposure of the previously conditioned tissue to a solution of sodium hydroxide at a mold concentration of 0.05 - 2 M, using 100 mL per gram of dry tissue, with efficient magnetic agitation and for a time of 2-6 hours, in order to remove surface proteins and leave collagen fibers exposed;
b1) carry out a series of final washes with distilled water, which allow neutralizing the pH of the tissue to a pH value between 7-8, before proceeding with the process.
c) Extraction where enzymatic hydrolysis is performed by subjecting the tissue to an acid solution of acetic acid at a molar concentration of 0.02 - 0.5 M and using 450 L per kg of dry tissue, together with 200 g of pepsin per kg of dry tissue (2.27% pepsin in 0.5 M acetic acid) to promote a faster solubilization of collagen and the specific and controlled removal of the terminal carboxyl and amino groups of the molecule; where the extraction is carried out over a period of three days by adding intermediate acid solution, starting with 200 mL of acetic acid per g of dry tissue and after 48 hours 250 mL of acetic acid per g of dry tissue are added, to promote the development of the reaction and where the residues of non-collagenous tissue structures are removed by means of filtration and the process continues.
d) Precipitation, where the resulting collagen solution (450 mL of solution per g of dry tissue) is brought to a high salt concentration by adding sodium chloride at a rate of 58.44 g of sodium chloride per L of collagen solution, the solution is homogenized with magnetic stirring and the ionic interaction of the salt with the collagen molecules is allowed to generate their precipitation in a time of about 2-6 hours. The resulting solution is sieved to a particle size between 125-850 µm; wherein the fibers recovered from the sieve are solubilized again in an acetic acid solution using 350 mL of acetic acid per g of dry tissue;
e) Dialysis, where in order to purify the solution from the excess of salt present in the collagen solution, a dynamic dialysis system is employed by means of which collagen is placed inside a porous membrane with a pore size of between 12-14 kD that allows the expulsion of impurities when introduced into a dialysis buffer consisting of a solution with a low concentration of acid acetic from 0.02 - 0.5 M; where the exchange of salt molecules between both solutions originates from their concentration differential, from the collagen solution with a salt concentration of 1 - 2 M towards the acetic acid solution without the presence of salts and is accelerated by the contact surface and the buffer flow; this stage lasts three to four days, during which the buffer is kept in recirculation and is changed after 48 hours; the conductivity of both solutions is also monitored and the process is stopped when collagen reaches the same conductivity as the buffer at the beginning of the stage of a value between 0.20 -0.28 mS/cm.
f) Lyophilization; where the purified collagen solution is subjected to a lyophilization cycle to concentrate the collagen fibers, favoring their preservation; in order to achieve this, the solution is frozen to -40°C and subsequently subjected to a vacuum pressure of 0.04 - 0.2 mbar (4 - 20 Pa) for a period of two days, during which water and solvents are removed in steam form drying the collagen without damaging the fibers; where the resulting collagen is weighed and distributed according to what is required in the next step.
g) Molding or second lyophilization, where depending on the application and expected function, a concentration of between 2.5-10 mg/mL is chosen and collagen is solubilized again in an acetic acid solution; where between 2.5 - 10 mg of collagen per mL of acetic acid with a molarity of between 0.02 - 0.5 M are used; once solubilized, collagen is placed in molds that allow to generate the desired structure; once again, the solution is lyophilized at a temperature of -40°C and a vacuum pressure of between 0.04 - 0.2 mbar (4 -20 Pa), with the exception that a controlled freezing is carried out (which consists of removing heat by gradually lowering the temperature of the plate with which the mold and collagen solution are in contact, allowing the water and acetic acid crystals to be uniform and varied, accommodating the fibers) to generate an estimated average pore size;
h) crosslinking, where the collagen pieces are subjected to a formaldehyde vapor atmosphere in a crosslinking apparatus, which allows the exposure time to be set between 1 - 60 minutes and the concentration of the reagent vapor cloud between 0.2 - 1.6 ppm resulting in a controlled crosslinking that allows reinforcing the bond between fibers that provides better physical properties to the structure.

In one of the modalities of the invention, the process also includes the stage of:
i) Pressing (or additional stage according to the application), where, in relation to the application and function to be performed by the product, where the collagen structure is subjected to a determined mechanical force of 400 - 5000 N to compact its dimensions to a desired value from 0.01 - 10 mm and increase its fibrillar density.

The human collagen structures obtained with controlled characteristics are type I human collagen, with a yield of 35%, preserving structural integrity and with purity ≥ 95%.

The human collagen structures obtained by the described process are also characterized in that they allow the cultivation and co-cultivation of primary and/or line human cells, and they function as a deposit of growth factors, proteins and exosomes, for their possible use in regenerative therapy.

In order to better understand the characteristics of the present invention, the present description is accompanied, as an integral part thereof, by the drawings with an illustrative but non-limiting nature, which are described below.

### BRIEF DESCRIPTION OF THE FIGURES

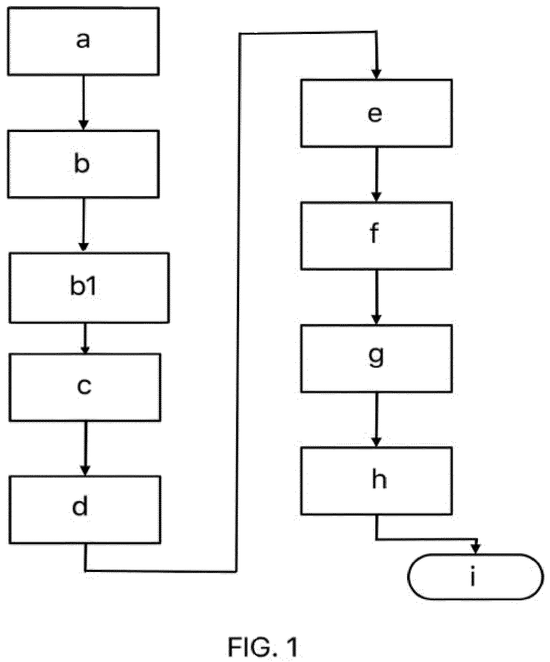

Figure 1 shows a flow chart of the novel process for the production of human collagen structures with controlled characteristics, in accordance with the present invention.

For a better understanding of the invention, there will be a detailed description of some of its modalities, shown in the drawings that are attached to this description for illustrative but not limiting purposes.

### DETAILED DESCRIPTION OF THE INVENTION

The characteristic details of the novel method for producing human collagen structures with controlled characteristics are clearly shown in the following description and in the attached illustrative drawings, which provide reference signs to indicate the same steps. During the development of the novel process for the production of human collagen structures with controlled characteristics, a series of problems arose that were gradually resolved until the process was standardized to obtain type I human collagen structures with controlled characteristics, with a yield of 35%, preserving structural integrity and with purity ≥ 95%.

The process to reduce the particle size of tissues was time consuming and involved a lot of manipulation. Besides that irregular pieces were obtained. It was solved by choosing to process the tissue in grinding equipment that would allow obtaining a paste from the tissue, without applying too much mechanical stress that would degrade its structure.

In order to maintain a low temperature during the process and to avoid the degradation of the extracted collagen fibers, a refrigerator with internal connection was adapted for the equipment used throughout the process.

Due to the amount of solution and the size of the fibers obtained after fractional precipitation, a sieving separation was implemented, which reduced the operation time and eliminated the need to acquire a larger and more specialized equipment to carry out separation by centrifugation of larger volumes of solution, resulting in only a 4% loss of fibers.

To enhance the mechanical performance and resistance to enzymatic degradation of the collagen structures and enable the final structure to adequately perform its intended function, chemical crosslinking of the structure was proposed; this operation falls within the field of bioconjugation.

In this context, chemical crosslinking should be understood as the creation of covalent bonds between two or more molecules. When carrying out this process, the result should be a structure with greater mechanical resistance, greater resistance to deformation, longer enzymatic degradation time and greater absorption. Different chemical substances that could achieve this effect were investigated and it was decided to try glutaraldehyde and formaldehyde.

Firstly, a solution of water and glutaraldehyde was used to immerse the structure in it. This method was unfeasible due to the delicate nature of the structure, which prevented the overall shape from being maintained, although it mainly affected the thickness and ductility of the material, making it highly brittle. Glutaraldehyde was simply too aggressive on the collagen structure, collapsing it while crosslinking it. The same technique was tried with a solution of water and formaldehyde, but the results were still unacceptable.

Afterwards, the decision was made to generate a gaseous medium in which the collagen structures were placed to interact at the molecular level with the formaldehyde gas. This medium or atmosphere was created simply by allowing the mixture of water and formaldehyde to remain for some time in a closed container at room temperature. The aqueous solution, due to the evaporation that naturally occurs at room temperature, eventually saturated the volume of the container. After a specified time, the container was opened and the collagen structures were introduced. Since the structures were inside the container, a specific time began to be counted. This method was equally unfeasible due to several factors: on the one hand, the water and formaldehyde solution did not evaporate in a determinable or controllable manner. In addition, when opening the container to introduce the collagen structures, the equilibrium reached by the atmosphere inside was disturbed.

This alteration could not be easily determined or measured. Ultimately, this method could not reliably generate repeatable results.

It was assumed that using mixtures of formaldehyde with some other medium (such as water), would make it more difficult to control the amount of gas generated. Therefore, the decision was made to produce the purest gas possible. It was demonstrated that it was feasible to heat paraformaldehyde until reaching the sublimation temperature and generate formaldehyde gas. Once the gas has been produced, it should be passed to another closed chamber where the collagen structures had previously been placed on a support that would allow homogeneous interaction with the gas. It was also decided that a certain amount of vacuum be produced in the chamber to minimize the effect that atmospheric air might have on the crosslinking process. Another issue that had to be resolved was how to stop the process abruptly in order to control both the start and the end of the crosslinking. To achieve this, a method was conceived to include a medical-grade air intake that would serve to purge the system and remove the formaldehyde.

By removing the formaldehyde, the interaction between collagen molecules and formaldehyde molecules is stopped, effectively stopping the crosslinking process. All of these design requirements were generated based on observation and experiments as described in the previous paragraphs. So the final design of the apparatus is the result of an experimental process and it is also the solution that allows collagen structures to be achieved with the desired physical, mechanical, chemical and biological compatibility characteristics.

According to figure 1, the novel process for the production of human collagen structures with controlled characteristics, in accordance with the present invention, consists of the following stages:
a) Tissue conditioning, where tissues or fluids that are not of interest for the process are removed and it is reduced to a particle size of 0.5 to 1 mm in a uniform way, in order to achieve a better interaction between solutions and tissue in the subsequent steps.
b) Pre-treatment
   Exposure of the previously conditioned tissue to a solution of sodium hydroxide at a mold concentration of 0.05 - 2 M, using 100 mL per gram of dry tissue, with efficient magnetic agitation and for a time of 2-6 hours, in order to remove surface proteins and leave collagen fibers exposed;
b1) carry out a series of final washes with distilled water, which allow neutralizing the pH of the tissue to a pH value between 7-8, before proceeding with the process.
c) Extraction where enzymatic hydrolysis is performed by subjecting the tissue to an acid solution of acetic acid at a molar concentration of 0.02 - 0.5 M and using 450 L per kg of dry tissue, together with 200 g of pepsin per kg of dry tissue (2.27% pepsin in 0.5 M acetic acid) to promote a faster solubilization of collagen and the specific and controlled removal of the terminal carboxyl and amino groups of the molecule; where the extraction is carried out over a period of three days by adding intermediate acid solution, starting with 200 mL of acetic acid per g of dry tissue and after 48 hours 250 mL of acetic acid per g of dry tissue are added, to promote the development of the reaction and where the residues of non-collagenous tissue structures are removed by means of filtration and the process continues.
d) Precipitation, where the resulting collagen solution (450 mL of solution per g of dry tissue) is brought to a high salt concentration by adding sodium chloride at a rate of 58.44 g of sodium chloride per L of collagen solution, the solution is homogenized with magnetic stirring and the ionic interaction of the salt with the collagen molecules is allowed to generate their precipitation in a time of about 2-6 hours. The resulting solution is sieved to a particle size between 125-850 µm; wherein the fibers recovered from the sieve are solubilized again in an acetic acid solution using 350 mL of acetic acid per g of dry tissue;
e) Dialysis, where in order to purify the solution from the excess of salt present in the collagen solution, a dynamic dialysis system is employed by means of which collagen is placed inside a porous membrane with a pore size of between 12-14 kD that allows the expulsion of impurities when introduced into a dialysis buffer consisting of a solution with a low concentration of acid acetic from 0.02 - 0.5 M; where the exchange of salt molecules between both solutions originates from their concentration differential, from the collagen solution with a salt concentration of 1 - 2 M towards the acetic acid solution without the presence of salts and is accelerated by the contact surface and the buffer flow; this stage lasts three to four days, during which the buffer is kept in recirculation and is changed after 48 hours; the conductivity of both solutions is also monitored and the process is stopped when collagen reaches the same conductivity as the buffer at the beginning of the stage of a value between 0.20 -0.28 mS/cm.
f) Lyophilization; where the purified collagen solution is subjected to a lyophilization cycle to concentrate the collagen fibers, favoring their preservation; in order to achieve this, the solution is frozen to -40°C and subsequently subjected to a vacuum pressure of 0.04 - 0.2 mbar (4 - 20 Pa) for a period of two days, during which water and solvents are removed in vapor form drying the collagen without damaging the fibers; where the resulting collagen is weighed and distributed according to what is required in the next step.
g) Molding or second lyophilization, where depending on the application and expected function, a concentration of between 2.5-10 mg/mL is chosen and collagen is solubilized again in an acetic acid solution; where between 2.5 - 10 mg of collagen per mL of acetic acid with a molarity of between 0.02 - 0.5 M are used; once solubilized, collagen is placed in molds that allow to generate the desired structure; once again, the solution is lyophilized at a temperature of -40°C and a vacuum pressure of between 0.04 - 0.2 mbar (4 -20 Pa), with the exception that a controlled freezing is carried out (which consists of removing heat by gradually lowering the temperature of the plate with which the mold and collagen solution are in contact, allowing the water and acetic acid crystals to be uniform and varied, accommodating the fibers) to generate an estimated average pore size;
h) crosslinking, where the collagen pieces are subjected to a formaldehyde vapor atmosphere in a crosslinking apparatus, which allows the exposure time to be set between 1 - 60 minutes and the concentration of the reagent vapor cloud between 0.2 - 1.6 ppm resulting in a controlled crosslinking that allows reinforcing the bond between fibers that provides better physical properties to the structure.

In one of the modalities of the invention, the process also includes the stage of:
i) Pressing (or additional stage according to the application), where, in relation to the application and function to be performed by the product, where the collagen structure is subjected to a determined mechanical force of 400 - 5000 N to compact its dimensions to a desired value from 0.01 - 10 mm and increase its fibrillar density.

With the production process, it has been possible to use of an available, accessible raw material with the highest degree of biocompatibility; obtaining functional and structurally intact collagen, with profitable extraction yields through a compatible process for industrial production conditions. In addition, said process allows the elaboration of collagen structures with adjustable physical characteristics for a wide range of biological applications, by means of simple and low-cost techniques that allow the optimization of collagen concentration in the structures; such characteristics include the dimensions of the structure, fibrillar density, porosity and pore size, which strongly influence cell behavior. Likewise, it was possible to increase the mechanical performance of the structures, with a precise and controlled technique, without affecting the integrity, functionality, or biological safety of collagen.

The human collagen structures obtained by the described process are also characterized in that they allow the cultivation and co-cultivation of primary and/or line human cells, and they function as a deposit of growth factors, proteins and exosomes, for their possible use in regenerative therapy.

The invention has been sufficiently described so that a person with average knowledge in the field can reproduce and obtain the results mentioned in the present invention.

## Claims

1. A method for producing human collagen structures with controlled characteristics, **characterized by** comprising the following stages:
a) Tissue conditioning, where tissues or fluids that are not of interest for the process are removed and it is reduced to a particle size of 0.5 to 1 mm in a uniform way, in order to achieve a better interaction between solutions and tissue in the subsequent steps.
b) Pre-treatment
Exposure of the previously conditioned tissue to a solution of sodium hydroxide at a mold concentration of 0.05 - 2 M, using 100 mL per gram of dry tissue, with efficient magnetic agitation and for a time of 2-6 hours, in order to remove surface proteins and leave collagen fibers exposed;
b1) carry out a series of final washes with distilled water, which allow neutralizing the pH of the tissue to a pH value between 7-8, before proceeding with the process.
c) Extraction where enzymatic hydrolysis is performed by subjecting the tissue to an acid solution of acetic acid at a molar concentration of 0.02 - 0.5 M and using 450 L per kg of dry tissue, together with 200 g of pepsin per kg of dry tissue (2.27% pepsin in 0.5 M acetic acid) to promote a faster solubilization of collagen and the specific and controlled removal of the terminal carboxyl and amino groups of the molecule; where the extraction is carried out over a period of three days by adding intermediate acid solution, starting with 200 mL of acetic acid per g of dry tissue and after 48 hours 250 mL of acetic acid per g of dry tissue are added, to promote the development of the reaction and where the residues of non-collagenous tissue structures are removed by means of filtration and the process continues.
d) Precipitation, where the resulting collagen solution (450 mL of solution per g of dry tissue) is brought to a high salt concentration by adding sodium chloride at a rate of 58.44 g of sodium chloride per L of collagen solution, the solution is homogenized with magnetic stirring and the ionic interaction of the salt with the collagen molecules is allowed to generate their precipitation in a time of about 2-6 hours. The resulting solution is sieved to a particle size between 125-850 µm; wherein the fibers recovered from the sieve are solubilized again in an acetic acid solution using 350 mL of acetic acid per g of dry tissue;
e) Dialysis, where in order to purify the solution from the excess of salt present in the collagen solution, a dynamic dialysis system is employed by means of which collagen is placed inside a porous membrane with a pore size of between 12-14 kD that allows the expulsion of impurities when introduced into a dialysis buffer consisting of a solution with a low concentration of acid acetic from 0.02 - 0.5 M; where the exchange of salt molecules between both solutions originates from their concentration differential, from the collagen solution with a salt concentration of 1 - 2 M towards the acetic acid solution without the presence of salts and is accelerated by the contact surface and the buffer flow; this stage lasts three to four days, during which the buffer is kept in recirculation and is changed after 48 hours; the conductivity of both solutions is also monitored and the process is stopped when collagen reaches the same conductivity as the buffer at the beginning of the stage of a value between 0.20 -0.28 mS/cm.
f) Lyophilization; where the purified collagen solution is subjected to a lyophilization cycle to concentrate the collagen fibers, favoring their preservation; in order to achieve this, the solution is frozen to -40°C and subsequently subjected to a vacuum pressure of 0.04 - 0.2 mbar (4 - 20 Pa) for a period of two days, during which water and solvents are removed in vapor form drying the collagen without damaging the fibers; where the resulting collagen is weighed and distributed according to what is required in the next step.
g) Molding or second lyophilization, where depending on the application and expected function, a concentration of between 2.5-10 mg/mL is chosen and collagen is solubilized again in an acetic acid solution; where between 2.5 - 10 mg of collagen per mL of acetic acid with a molarity of between 0.02 - 0.5 M are used; once solubilized, collagen is placed in molds that allow to generate the desired structure; once again, the solution is lyophilized at a temperature of -40°C and a vacuum pressure of between 0.04 - 0.2 mbar (4 -20 Pa), with the exception that a controlled freezing is carried out (which consists of removing heat by gradually lowering the temperature of the plate with which the mold and collagen solution are in contact, allowing the water and acetic acid crystals to be uniform and varied, accommodating the fibers) to generate an estimated average pore size;
h) crosslinking, where the collagen pieces are subjected to a formaldehyde vapor atmosphere in a crosslinking apparatus, which allows the exposure time to be set between 1 - 60 minutes and the concentration of the reagent vapor cloud between 0.2 - 1.6 ppm resulting in a controlled crosslinking that allows reinforcing the bond between fibers that provides better physical properties to the structure.

2. The method for producing human collagen structures with controlled characteristics, according to claim 1, **characterized in that** it also comprises the stage of:
i) Pressing (or additional stage according to the application), where, in relation to the application and function to be performed by the product, where the collagen structure is subjected to a determined mechanical force of 400 - 5000 N to compact its dimensions to a desired value from 0.01 - 10 mm and increase its fibrillar density.

3. The method for producing human collagen structures with controlled characteristics, according to the previous claims, **characterized in that** collagen has a purity ≥ 95%.

## Patentansprüche

1. Verfahren zur Herstellung von menschlichen Kollagenstrukturen mit kontrollierten Eigenschaften, **dadurch gekennzeichnet, dass** es die folgenden Stufen umfasst:
a) Konditionieren von Gewebe, wobei Gewebe oder Flüssigkeiten, die für den Prozess nicht von Interesse sind, entfernt und auf eine Partikelgröße von 0,5 bis 1 mm gleichmäßig verkleinert werden, um in den nachfolgenden Schritten eine bessere Wechselwirkung zwischen Lösungen und Gewebe zu erzielen;
b) Vorbehandeln
Aussetzen des zuvor konditionierten Gewebes einer Natriumhydroxidlösung mit einer molaren Konzentration von 0,05-2 M unter Verwendung von 100 ml pro Gramm Trockengewebe, unter effizientem magnetischem Rühren und für eine Dauer von 2-6 Stunden, um Oberflächenproteine zu entfernen und Kollagenfasern freizulegen;
b1) Durchführen einer Reihe von abschließenden Spülungen mit destilliertem Wasser, wodurch der pH-Wert des Gewebes auf einen Wert zwischen 7-8 neutralisiert wird, bevor mit dem Verfahren fortgefahren wird;
c) Extrahieren, wobei die enzymatische Hydrolyse durchgeführt wird, indem das Gewebe einer Säurelösung von Essigsäure bei einer molaren Konzentration von 0,02-0,5 M ausgesetzt wird und unter Verwendung von 450 I pro kg Trockengewebe zusammen mit 200 g Pepsin pro kg Trockengewebe (2,27 % Pepsin in 0,5 M Essigsäure), um eine schnellere Solubilisierung des Kollagens und die spezifische und kontrollierte Entfernung der terminalen Carboxyl- und Aminogruppen des Moleküls zu fördern; wobei das Extrahieren über einen Zeitraum von drei Tagen durch Zugabe von intermediären Säurelösungen durchgeführt wird, beginnend mit 200 ml Essigsäure pro g Trockengewebe, und nach 48 Stunden 250 ml Essigsäure pro g Trockengewebe zugegeben werden, um die Entwicklung der Reaktion zu fördern, und wobei die Rückstände von nichtkollagenen Gewebestrukturen mittels Filtration entfernt werden und der Prozess fortgesetzt wird;
d) Präzipitieren, wobei die resultierende Kollagenlösung (450 ml Lösung pro g Trockengewebe) durch Zugabe von Natriumchlorid in einer Menge von 58,44 g Natriumchlorid pro Liter Kollagenlösung auf eine hohe Salzkonzentration gebracht wird, die Lösung unter magnetischem Rühren homogenisiert wird und die ionische Wechselwirkung des Salzes mit den Kollagenmolekülen innerhalb von etwa 2-6 Stunden zu deren Präzipitation führt; die resultierende Lösung wird auf eine Partikelgröße zwischen 125-850 µm gesiebt; wobei die aus dem Sieb zurückgewonnenen Fasern erneut in einer Essigsäurelösung unter Verwendung von 350 ml Essigsäure pro g Trockengewebe solubilisiert werden;
e) Dialysieren, wobei, um die Lösung von dem in der Kollagenlösung vorhandenen Salzüberschuss zu reinigen, ein dynamisches Dialysesystem verwendet wird, mit dessen Hilfe das Kollagen in eine poröse Membran mit einer Porengröße zwischen 12-14 kD eingebracht wird, die das Austreiben von Verunreinigungen ermöglicht, wenn sie in einen Dialysepuffer eingeführt wird, der aus einer Lösung mit einer niedrigen Essigsäurekonzentration von 0,02-0,5 M besteht; wobei der Austausch von Salzmolekülen zwischen beiden Lösungen aufgrund ihrer Konzentrationsdifferenz stattfindet, und zwar von der Kollagenlösung mit einer Salzkonzentration von 1-2 M bis zur Essigsäurelösung ohne Salze, und durch die Kontaktfläche und den Pufferstrom beschleunigt wird; diese Stufe dauert drei bis vier Tage, in denen der Puffer in Umwälzung gehalten und nach 48 Stunden gewechselt wird; die Leitfähigkeit beider Lösungen wird ebenfalls überwacht und der Prozess wird gestoppt, wenn das Kollagen die gleiche Leitfähigkeit wie der Puffer zu Beginn der Stufe erreicht, d. h. einen Wert zwischen 0,20-0,28 mS/cm;
f) Lyophilisieren; wobei die gereinigte Kollagenlösung einem Lyophilisierungszyklus unterzogen wird, um die Kollagenfasern zu konzentrieren, wodurch ihre Konservierung begünstigt wird; dazu wird die Lösung auf -40 °C eingefroren und anschließend zwei Tage lang einem Vakuumdruck von 0,04-0,2 mbar (4-20 Pa) ausgesetzt, bei dem Wasser und Lösungsmittel in Dampfform entfernt werden, wodurch das Kollagen getrocknet wird, ohne die Fasern zu beschädigen; wobei das resultierende Kollagen gewogen und entsprechend den Anforderungen des nächsten Schritts verteilt wird;
g) Formgeben oder zweites Lyophilisieren, wobei je nach Anwendung und erwarteter Funktion eine Konzentration zwischen 2,5-10 mg/ml gewählt wird und das Kollagen erneut in einer Essigsäurelösung gelöst wird; wobei zwischen 2,5-10 mg Kollagen pro ml Essigsäure bei einer Molarität zwischen 0,02 und 0,5 M verwendet werden; nach dem Solubilisieren, wird das Kollagen in Formen eingebracht, die es ermöglichen, die gewünschte Struktur zu erzeugen; die Lösung wird erneut bei einer Temperatur von -40 °C und einem Vakuumdruck von 0,04-0,2 mbar (4-20 Pa) lyophilisiert, mit der Ausnahme, dass ein kontrolliertes Einfrieren durchgeführt wird (das darin besteht, die Wärme zu entziehen, indem die Temperatur der Platte, mit der die Form und die Kollagenlösung in Kontakt stehen, allmählich gesenkt wird, wodurch die Wasser- und Essigsäurekristalle gleichmäßig und vielfältig werden und die Fasern aufnehmen), um eine geschätzte durchschnittliche Porengröße zu erzeugen;
h) Vernetzen, wobei die Kollagenstücke in einem Vernetzungsgerät einer Formaldehyd-Dampfatmosphäre ausgesetzt werden, wobei die Einwirkzeit zwischen 1-60 Minuten und die Konzentration der Reagenzdampfwolke zwischen 0,2-1,6 ppm eingestellt werden kann, was zu einer kontrollierten Vernetzung führt, die eine Verstärkung der Bindung zwischen den Fasern ermöglicht und der Struktur bessere physikalische Eigenschaften verleiht.

2. Verfahren zur Herstellung von menschlichen Kollagenstrukturen mit kontrollierten Eigenschaften gemäß Anspruch 1, **dadurch gekennzeichnet, dass** es auch die folgende Stufe umfasst:
i) Pressen (oder zusätzliche Stufe entsprechend der Anwendung), wobei je nach Anwendung und Funktion des Produkts die Kollagenstruktur einer bestimmten mechanischen Kraft von 400-5000 N ausgesetzt wird, um ihre Abmessungen auf einen gewünschten Wert von 0,01-10 mm zu verdichten und ihre Faserdichte zu erhöhen.

3. Verfahren zur Herstellung von menschlichen Kollagenstrukturen mit kontrollierten Eigenschaften gemäß den vorhergehenden Ansprüchen, **dadurch gekennzeichnet, dass** das Kollagen eine Reinheit von ≥ 95 % aufweist.

## Revendications

1. Procédé pour produire des structures de collagène humain ayant des caractéristiques contrôlées, **caractérisé en ce qu'**il comprend les étapes suivantes :
a) le conditionnement du tissu, où les tissus ou les fluides qui ne présentent pas d'intérêt pour le processus sont éliminés et réduit à une taille de particule de 0,5 à 1 mm de manière uniforme, afin d'obtenir une meilleure interaction entre les solutions et le tissu dans les étapes suivantes ;
b) le pré-traitement L'exposition du tissu préalablement conditionné à une solution d'hydroxyde de sodium à une concentration de moule de 0,05 à 2 M, en utilisant 100 ml par gramme de tissu sec, avec une agitation magnétique efficace et pendant une durée de 2 à 6 heures, afin d'éliminer les protéines de surface et de laisser les fibres de collagène exposées ;
b1) la réalisation d'une série de lavages finaux avec de l'eau distillée, qui permettent de neutraliser le pH du tissu à une valeur de pH comprise entre 7 et 8, avant de poursuivre le processus ;
c) l'extraction où l'hydrolyse enzymatique est réalisée en soumettant le tissu à une solution acide d'acide acétique à une concentration molaire de 0,02 à 0,5 M et en utilisant 450 L par kg de tissu sec, avec 200 g de pepsine par kg de tissu sec (2,27 % de pepsine dans de l'acide acétique à 0,5 M) pour favoriser une solubilisation plus rapide du collagène et l'élimination spécifique et contrôlée des groupes carboxyle et aminé terminaux de la molécule ; où l'extraction est réalisée sur une période de trois jours par l'ajout d'une solution acide intermédiaire, en commençant avec 200 ml d'acide acétique par g de tissu sec et, après 48 heures, 250 ml d'acide acétique par g de tissu sec sont ajoutés, pour favoriser le développement de la réaction et où les résidus de structures tissulaires non collagéniques sont éliminés par filtration et le processus se poursuit ;
d) la précipitation, où la solution de collagène obtenue (450 ml de solution par g de tissu sec) est amenée à une concentration élevée en sel par l'ajout de chlorure de sodium à raison de 58,44 g de chlorure de sodium par L de solution de collagène, la solution est homogénéisée par agitation magnétique et l'interaction ionique du sel avec les molécules de collagène est autorisée à générer leur précipitation dans un délai d'environ 2 à 6 heures ; la solution obtenue est tamisée pour obtenir une taille de particule comprise entre 125 et 850 µm ; dans lequel les fibres récupérées du tamis sont à nouveau solubilisées dans une solution d'acide acétique en utilisant 350 ml d'acide acétique par g de tissu sec ;
e) la dialyse, où afin de purifier la solution de l'excès de sel présent dans la solution de collagène, un système de dialyse dynamique est utilisé par le biais duquel le collagène est placé à l'intérieur d'une membrane poreuse avec une taille de pore comprise entre 12 et 14 kD et qui permet l'expulsion des impuretés lorsqu'elle est introduite dans un tampon de dialyse constitué d'une solution à faible concentration d'acide acétique de 0,02 à 0,5 M ; où l'échange de molécules de sel entre les deux solutions provient de leur différence de concentration, de la solution de collagène avec une concentration de sel de 1 à 2 M vers la solution d'acide acétique sans la présence de sels et est accélérée par la surface de contact et le flux de tampon ; cette étape dure trois à quatre jours, pendant laquelle le tampon est maintenu en recirculation et est changé après 48 heures ; la conductivité des deux solutions est également surveillée et le processus est arrêté lorsque le collagène atteint la même conductivité que le tampon au début de l'étape d'une valeur comprise entre 0,20 et 0,28 mS/cm ;
f) la lyophilisation ; où la solution de collagène purifiée est soumise à un cycle de lyophilisation pour concentrer les fibres de collagène, favorisant leur préservation ; à cette fin, la solution est congelée à -40 °C et ensuite soumise à une pression sous vide de 0,04 à 0,2 mbar (4 à 20 Pa) pendant une période de deux jours, au cours de laquelle l'eau et les solvants sont éliminés sous forme de vapeur en séchant le collagène sans endommager les fibres ; où le collagène obtenu est pesé et réparti en fonction des besoins de l'étape suivante ;
g) le moulage ou seconde lyophilisation, où en fonction de l'application et de la fonction attendue, une concentration comprise entre 2,5 et 10 mg/ml est choisie et le collagène est à nouveau solubilisé dans une solution d'acide acétique ; où entre 2,5 et 10 mg de collagène par ml d'acide acétique d'une molarité comprise entre 0,02 et 0,5 M son utilisé ; une fois solubilisé, le collagène est placé dans des moules qui permettent de générer la structure souhaitée ; une fois de plus, la solution est lyophilisée à une température de -40 °C et à une pression de vide comprise entre 0,04 et- 0,2 mbar (4 -20 Pa), à l'exception d'une congélation contrôlée (qui consiste à éliminer la chaleur en abaissant progressivement la température de la plaque avec laquelle le moule et la solution de collagène sont en contact), permettant aux cristaux d'eau et d'acide acétique d'être uniformes et variés, accueillant les fibres) pour générer une estimation de la taille moyenne des pores ;
h) la réticulation, où les morceaux de collagène sont soumis à une atmosphère de vapeur de formaldéhyde dans un appareil de réticulation, qui permet de régler le temps d'exposition entre 1 et 60 minutes et la concentration du nuage de vapeur du réactif entre 0,2 et 1,6 ppm, ce qui entraîne une réticulation contrôlée qui permet de renforcer la liaison entre les fibres qui fournit de meilleures propriétés physiques à la structure.

2. Procédé pour produire des structures de collagène humain ayant des caractéristiques contrôlées, selon la revendication 1, **caractérisé en ce qu'**il comprend également l'étape :
i) le pressage (ou étape supplémentaire selon l'application), où, par rapport à l'application et à la fonction qui doit être réalisé par le produit, où la structure de collagène est soumise à une force mécanique déterminée de 400 à 5000 N pour compacter ses dimensions à une valeur souhaitée de 0,01 à 10 mm et augmenter sa densité fibrillaire.

3. Procédé pour produire des structures de collagène humain ayant des caractéristiques contrôlées, selon les revendications précédentes, **caractérisé en ce que** le collagène a une pureté ≥ 95 %.
